# EUROPEAN PATENT SPECIFICATION

(11) **EP 0 510 187 B1**
(45) Date of publication and mention of the grant of the patent: **18.02.1998**
(21) Application number: 92902046.9
(22) Date of filing: 06.11.1991
(51) Int. Cl.: A61M 5/00

(54) **CATHETER ASSEMBLY HAVING SAFETY MEANS AND METHOD FOR USING SAME**
KATHETEREINRICHTUNG MIT SICHERHEITSELEMENT SOWIE VERFAHREN ZU DEREN GEBRAUCH
ENSEMBLE CATHETER POURVU D'UN ELEMENT DE SECURITE ET METHODE POUR SON UTILISATION

(30) Priority: 08.11.1990 US 610583
(43) Date of publication of application: 28.10.1992
(73) Proprietor: MBO LABORATORIES, INC., Carlisle, MA 07141 (US)
(72) Inventor: McCORMICK, William, Carlisle, MA 01741 (US); BLECHER, Jacob, B., Lexington, MA 02173 (US); O'DONNELL, Miles, C., Andover, MA 01810 (US)
(74) Representative: Harvey, David Gareth
(86) International application number: PCT/US91/08063
(87) International publication number: WO 92/08502

(56) References cited:
- EP-A- 0 281 421
- WO-A-88/07388
- WO-A-89/04183
- WO-A-89/10767
- WO-A-90/00075
- WO-A-91/01151
- DE-A- 3 808 688
- US-A- 3 709 223
- US-A- 4 935 012
- US-A- 4 943 281

## Description

### Background of the Invention

In recent years there has been considerable research and development with respect to single-use disposable needles or needle stylets for intravenous and like use to minimize the hazards of contamination from reused needles, as well as to obviate the time-consuming, labor intensive, and expensive necessity of effective cleaning, sterilization, and repackaging of reusable needles.

While single-use throwaway needles admirably eliminate the adverse aspects of cleaning of and contamination from reused needles, this has resulted in newer problems with respect to unintended access to and danger from the used throwaway needle. The hazards include inadvertent contact with or skin rupture from a previously used and contaminated needle during intended and proper handling thereof by healthcare personnel, as in collection and disposal thereof.

With increasing concerns of potentially debilitating or fatal infections of healthcare workers or others by accidental or even deliberate needlestick injuries from used intravenous needles variously contaminated as with tissue residue of patients with AIDS virus, hepatitis B virus, or other pathogens, a number of efforts have been made to devise means for shielding or otherwise rendering inaccessible a used needle.

In providing such protection against hazardous contact with a used needle, there is an inherent practical conflict with ease and economy of manufacture, and ease of needle usage, including insertion and withdrawal. In like manner, while a simplified shielding means may not adversely impact upon manufacture and use, sacrifice is made in convenience and reliability of contamination shielding.

Illustrative devices known in the art include that of European Patent Application 0314470, published May 3, 1989, owned by Menlo Care Inc. of Palo Alto, California. The same is intended for use only as an IV catheter placement system, including a laterally winged catheter insertion means and a completely separate extractor assembly into which the contaminated needle point is withdrawn after use. The several components are complex of fabrication and in use, and do not provide facile, reliable means for shielding a needle.

U.S. Patent 4,790,828 to Dombrowski et al deals with a self-capping needle wherein a needle cap is connected or tethered to the needle in a difficultly fabricated and handled assembly.

Other assemblies seeking to protect or shield a needle or stylet after use are typified by U.S. Patents 4,676,783 and 4,781,692 to Jagger et al, which while theoretically useful, present manufacture and manipulative difficulties.

The prior art is replete with medical needle devices arranged to guard against inadvertent needle stick injuries or contact with contaminated needles.

For example, US-A-4,935,012 discloses a needle supported in a barrel, over which a protective sleeve is slidable, the sleeve having a tip with a narrow bore through which the needle can emerge. In a first position of the sleeve, the needle tip is lodged safely within the bore. To use the needle, the sleeve is slid in a rearward direction relative to the needle point, whereby the needle is exposed. After use, the sleeve is returned toward and past the first position to a second position wherein the bore has disengaged from the needle tip, the latter now becoming lodged within the sleeve, rearwardly of the bore. Closure flaps are located at an entrance to the bore in an effort to ensure that the needle, once disengaged from the bore, cannot be manipulated to protrude again from the sleeve.

Somewhat similarly, DE-A-3,808,688 discloses a two-piece needle guard (2.1, 2.2). Guard piece 2.1 is a bellows-like sleeve and guard piece 2.2 joined thereto is a rigid tube pierced by a needle channel at a front end thereof. The needle is extended through the channel for injecting, and after use the needle guard is extended lengthwise along the needle until the needle tip clears the needle channel and enters the inside of guard piece 2.2. The guard is flexed slightly such that the needle point becomes lodged in an inside corner of the guard piece 2.2.

WO88/07387 shows a needle device wherein a proximal end of the needle is mounted in an end of a tube which is slidable within a second, larger tube. After an injection, the inner tube plus needle are telescoped within the outer tube, until the needle point is housed inside the outer tube; it is held in this position by friction between the two tubes.

WO89/04183 discloses a tubular sheath for receiving a used needle. The sheath externally has the general appearance of a test tube, but adjacent its open end there is a one-way entrance. Its bottom end has a small aperture through which a wire is threaded, the wire being connected to the needle. After using the needle, it is drawn backwardly into the sheath using the wire, and once its point passes the one-way entrance, the needle is trapped within the sheath.

EP-A-0,314,470 teaches a device in most relevant respects functionally and structurally equivalent to that shown in WO89/04183.

Taught in US-A-4,943,281 is a disposable syringe fitted with a needle and needle guard in the form of a protective bow. The bow has a pair of opposed limbs united by an arch of the bow, the arch being pierced to allow the needle to be advanced beyond the arch to an injection position. After injection, a needle retracting means returns the needle to a position in which the needle point is located within the passage pierced in the arch. Only the point region is guarded: the main length of the needle is exposed between the limbs of the bow.

EP-A-0,281,421 discloses a protectable needle for fitting to a syringe. The needle extends from a tubular housing some half the length of the needle, which at its proximal end is mounted via a luer-lock type of hub to the corresponding end of the housing. A tubular sleeve, also some half the length of the needle, is telescoped within the housing about the needle. After an injection, the sleeve is moved outwardly of the housing along the needle towards the distal end thereof. When it is fully extended, the sleeve latches to the housing and in this position the needle point is set back just inside the sleeve.

With the exception of US-A-4,943,281, the prior art documents just summarised above rely on generally tubular guard elements into which contaminated needles are retracted after injection. Although US-A-4,943,281 does not have a tubular guard element as such, its needle point is somewhat similarly protected when it is retracted into the confines of the passage through the arch.

The present Applicant fears that prior art arrangements as summarised above are not foolproof, and may potentially be less safe than desired. According to the Applicant's invention, in addition to a tubular needle guard there is a sprung "shutter" arrangement coupled to the needle guard. When the needle is retracted into the guard, the shutter closes across the open end of the guard, positively preventing further ejection of the needle from the guard tube.

WO90/00075 has a rather similar needle guard system, but the shutter arrangement taught here is configured such that the needle is readily reusable after suitable manipulation of the shutter arrangement. Reusability is contrary to the Applicant's intentions. The present shutter arrangement, as will be described in detail, is configured so as to make reuse virtually impossible.

It is an object of the present invention to provide a readily manufactured and easily used assembly in association with an IV needle whereby the needle is safely shielded for disposal in a reliable and virtually fail-proof manner.

The invention in its apparatus and method aspects is defined in appended claims 1 and 12 respectively. Preferred features of the apparatus are defined in claims 2 to 11.

In accordance with the invention, the safely disposable intravenous needle assembly includes several cooperatively associated components utilized in conjunction with a needle, namely: (1) guide means for suitable receiving a needle cannula or needle stylet, and within which guide means the used, contaminated needle is to be received for safety shielding after use; (2) manipulating means affixed to the needle to effect safe enclosure thereof; (3) interengageable means provided on the guide means and manipulating means to facilitate insertion and safe withdrawal of the needle in intravenous use.

Further, the guide means uniquely includes and carries a needle point blocking or shielding device automatically operative upon withdrawal of the used or contaminated needle into the guide means. The point shield may partake of several forms.

Attentively thereto, the guide means and manipulative means are interconnected by flexible and resilient means to facilitate unit-handled manufacture of the assembly as well as to control safe needle positioning within and without the guide means.

In a further form of the invention, there is provided a soft over-the-needle catheter device which is disposed on the needle during insertion and remains in place in the blood vessel after withdrawal of the needle. In like manner, other forms of this invention embrace utilization with blood collection systems and hypodermic needle syringe devices.

The guide means, of the invention, comprises a central tube having a bore or passageway therethrough for reception and sliding movement of a needle therein. The guide means further includes wing-like sections extending laterally from the central tube, which wings are preferably molded integrally with the central tube and are flexible with respect thereto. The operation of the device in shielding the needle after use will be set forth hereinafter, but it is noted that as part thereof, the needle will be fully withdrawn into the tubular passageway with no portion of the needle distal end projecting therefrom.

Means are provided to assure positive retention of the needle distal end within the guide member and protection from any hazard of an exposed needle tip. To this end, in a preferred form, the tubular member carries a guard device in the nature of a leaf spring which is mounted at its proximate end on the tubular member and at its distal end is provided with a needle blocking plate. The spring device as mounted on the tube is tensioned to project the blocking plate in front of the passageway at the tube distal end, but is unable to do so by projection of the needle therefrom prior to and in use, during which the blocking plate is spring-urged to bear against the needle.

The distal pointed end of the needle extends from the tube, and the rearward proximal portion of the needle carries the manipulating means, namely a base member receiving the needle therethrough to which it is affixed, whereby manual movement of the base member will also move the needle. As is evident, the base member and the tubular passageway are axially aligned, wherein the manipulating means is disposed rearwardly of the guide means.

Interconnection and separation limit means are provided between the guide means and the manipulating means in the form of a resilient loop or strap, which is preferably integrally formed therewith. Accordingly, the same serves as a restraint to rearward or separating movement of the manipulating member with respect to the guide member as well as enables the unit molding and handling of the guide member and manipulating member.

Interengagement means are provided in the nature of flanges on the base member of the manipulating member and on the flexible wings of the guide member, whereby upon flexure thereof, the interengaging means become substantially axially aligned. At such time, after the base member of the manipulating member is advanced initially to extend the needle point distally from the guide member, with the wings flexed together to guide the needle during insertion and to stabilize the assembly, upon engagement of the manipulating member with the guide member in this manner, the needle, guide member and manipulating member move as one unit in subcutaneously inserting the needlepoint.

After appropriate intravenous or other operations are performed through the needle, the needle is withdrawn from the tissue and safely secured. To this end, the flexible wings are held stationary and the guide member is held abutting the skin needle insertion site and the manipulating member is withdrawn in a proximate direction, thereby retracting the needle and needlepoint into the tubular bore of the guide member. As the needle tip passes fully into the bore, the restraint against the leaf spring blocking plate is eliminated, and the blocking plate snaps transversely over the tubular member to effectively seal the end of the bore and thereby preclude any likelihood of the contaminated needle tip from emerging. Other safety means are set forth, all blocking any emergence of the contaminated tip from the guide member.

Further, the needle cannot be fully withdrawn in a rearward direction from the bore inasmuch as the dimensioning of the interconnecting restraining loops is such that when manipulating member base member carrying the needle is in its most remote position from the guide member, the interconnecting loops have insufficient length to permit the needle to approach withdrawal from the guide member passageway. The used needle assembly may now safely be discarded in toto.

### Brief Description of the Drawings

The invention will be better understood when taken with the accompanying drawings, in which:
Fig. 1 is a top plan view of a preferred embodiment of the invention showing the needle assembly prior to use;
Fig. 2 is a plan view similar to Fig. 1 but wherein the needle has been advanced relative to the guide means;
Fig. 3 is a side elevation of an illustrative leaf spring of a needle blocking and securing means;
Fig. 3a is a perspective view of a modified leaf spring of a further form of needle blocking and securing means;
Fig. 4 is a side view taken on the lines 4-4 of Fig. 2; portions thereof being shown in section;
Fig. 5 is a frontal sectional view on the lines 5-5 as shown in Fig. 2.
Fig. 6 is a frontal sectional view as in Fig. 5 but with the wing sections of the guide member flexed to the guide member/manipulating member interengaging position;
Fig. 7 is a top plan view similar to Fig. 1 but with the manipulating member fully retracted in a proximal direction with respect to the guide member to enclose and shield the needle;
Fig. 8 is a side view taken on the lines 8-8 of Fig. 7; portions thereof being shown in section;
Fig. 9 is similar to Fig. 8 but wherein the guide member is of somewhat more flexible material;
Fig. 10 is a view similar to Fig. 9 showing a modification outside the scope of the invention;
Fig. 11 is a view similar to Fig. 2 but showing a further form of the invention;
Fig. 12 is a view similar to Fig. 2 but utilizing a catheter in conjunction with a needle; perspective view thereof with the wings flexed at the time of tissue penetration;
Fig. 13 is a perspective view of the device of Fig. 11 and Fig. 12 but with the wings returned to substantially planar position for needle withdrawal and shielding;
Fig. 14 is a perspective view thereof with the needle assembly withdrawn, the catheter remaining in situ, and the needle shielded;
Fig. 15 is a top plan view similar to Fig. 1 of a further modified form of the invention;
Fig. 16 is a top plan view similar to Fig. 15 of the further modified form of the invention in a different position;
Fig. 17 is a top plan view similar to Fig. 16 of the further modified form of the invention showing the used needle in a fully withdrawn position with the entire needle covered;
Fig. 18 is a top plan view similar to Fig. 17 of another modified form of the invention;
Fig. 19 is a top plan view similar to Fig. 2 of a fifth modified form of the invention adapted for blood collection;
Fig. 20 is a top plan view similar to Fig. 19 of the fifth modified form of the invention when in use for blood collection;
Fig. 21 is a top plan view of a sixth modified form of the invention for use in a hypodermic needle syringe configuration;
Fig. 22 is a top plan view similar to Fig. 1 of a seventh modified form of the invention wherein the interconnecting straps are detachably connected to the manipulating member;
Fig. 23 is a top plan view of yet another form of the invention wherein the wings and manipulating members are altered.
Fig. 24 is a fragmentary side elevation of a further form of needle assembly utilizing the spring of Fig. 3a;
Fig. 25 is an end sectional view taken on the line 25-25 of Fig. 24;
Fig. 26 is a fragmentary perspective view of the proximal end of the needle assembly without the spring;
Fig. 27 is a view similar to Fig. 24, but showing the retracted needle position and safety blocking thereof;
Fig. 28 is a view similar to Fig. 24, but illustrating a yet further modified needle assembly and safety guard spring;
Fig. 29 is a sectional end view taken on the lines 29-29 of Fig. 28; and,
Fig. 30 is a sectional end view of the needle assembly front section alone.

### Description of Preferred Embodiments

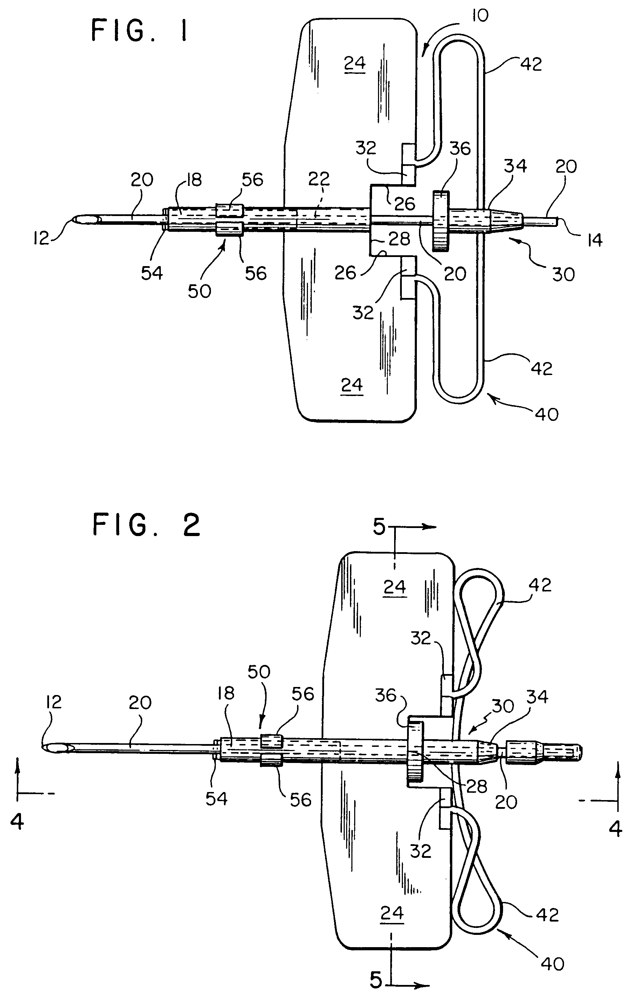

Referring to the drawings, Figs. 1 - 4 show a preferred embodiment of the invention incorporating the unique features and basic concepts thereof. The same embraces a guide member 10 for effecting primary shielding of needle 20, a manipulating member 30 disposed rearwardly of the guide member 10, and interconnecting means 40 between the guide member and the manipulating member, the cooperation of which will be set forth more fully hereinafter. Also, as further explained, the members 10, 30, and 40 are preferably molded, as by injection molding, as a single integral unit-handled assembly.

Thus, a generally conventional metal needle cannula 20 includes the usual beveled and sharpened tip 12 at its distal end and a remote proximal end 14 which is connected to medical tubing (not shown) in known manner. Alternatively, medical tubing could be affixed to the enlarged proximal end of manipulating member 30. Surrounding needle 20 is the guide member and primary needle shielding component 10 which includes central tubular portion 18 having a bore or passageway 22 therethrough within which is suitable received needle 20. Tubular member 18 is of extended length so as to embrace a significant length of needle 20 to adequately guide and support the same.

Formed integrally with tubular portion 18 and extending laterally thereof on either side are generally planar and flexible wing-like sections 24. The wings 24 are formed at 26 to define a pocket or recess 28, and the wing proximal portions thereadjacent are provided with upstanding lugs or flanges 32.

While needle 20 is normally free to slide within passageway 22 of guide member 10, the manipulating member 30 includes a tubular base member 34 which is bonded securely to the periphery of needle 20 near its proximal end so as to be rigid therewith, whereby manual movement of the base member 34 alone will effect like movement therewith of the needle.

Manipulating member 30 has at its distal end a radially enlarged flange 36 for interengaging cooperation with the flanges 32 of wings 24 when in mutually generally axially aligned condition, as set forth hereinafter.

Interconnecting and restraint means 40 between guide member 10 and manipulating member 30 are provided, namely flexible loop-like strap portions 42. The straps 42 as shown in Fig. 1 are respectively fixedly connected at their ends, as by integral molding, to wings 24 adjacent flanges 32 and to the base member 34. Irrespective of the particular points or nature of connection, the straps are precisely dimensioned with respect to the length of needle 20, guide member 10 and manipulating member 30 for a purpose noted hereinafter.

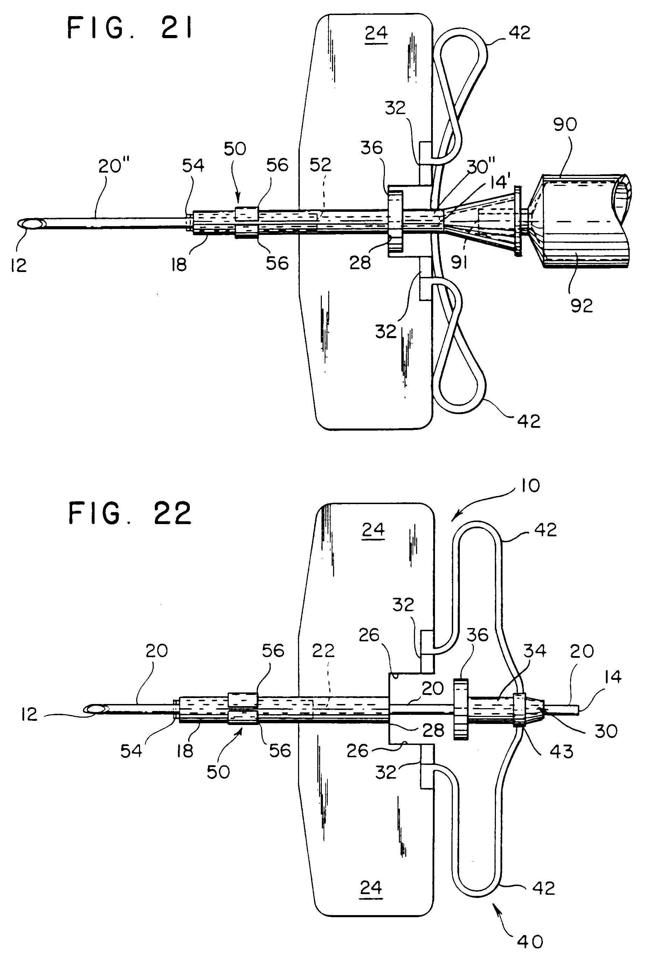

Another alternative form of the invention is illustrated in Fig. 22 in which the interconnecting and restraint means between guide member 10 and manipulating member 30 is formed integrally with only one of these two members. In Fig. 22, for example, the restraint means is formed integrally with guide member 10 and has a loop 43 at its proximal end that encircles the conically shaped proximal end of manipulating member 30. The loop is of such a diameter that it fits onto the proximal end of manipulating member 30 so that it cannot slide off the distal end of manipulating member 30 and thereby limits the separation of the manipulating member 30 from the guide member 10 during needle withdrawal in accordance with this invention, as set forth hereinafter.

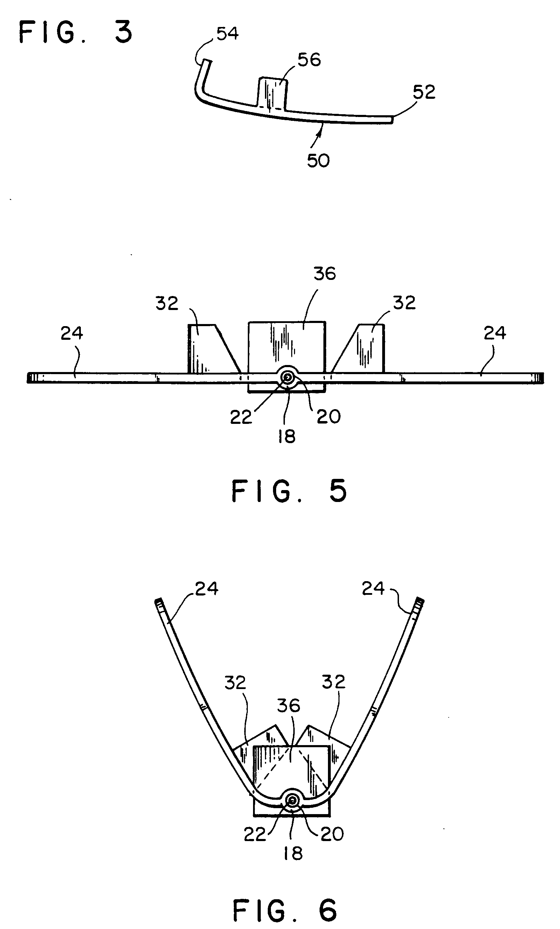

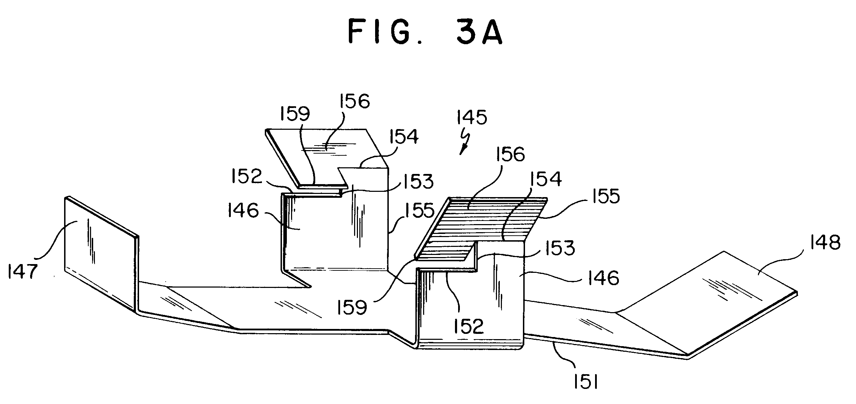

Importantly, in the embodiment of Figs. 1 - 4, tubular member 18 carries a needle point shield and guard device 50, seen alone in Fig. 3, which comprises in its neutral and relaxed position a curved leaf spring of steel or the like having an axially extending leg 52, a generally right-angled blocking plate 54, and a pair of clip legs 56 forming a gripping collar for securely holding the guard device 50 onto the guide member 10.

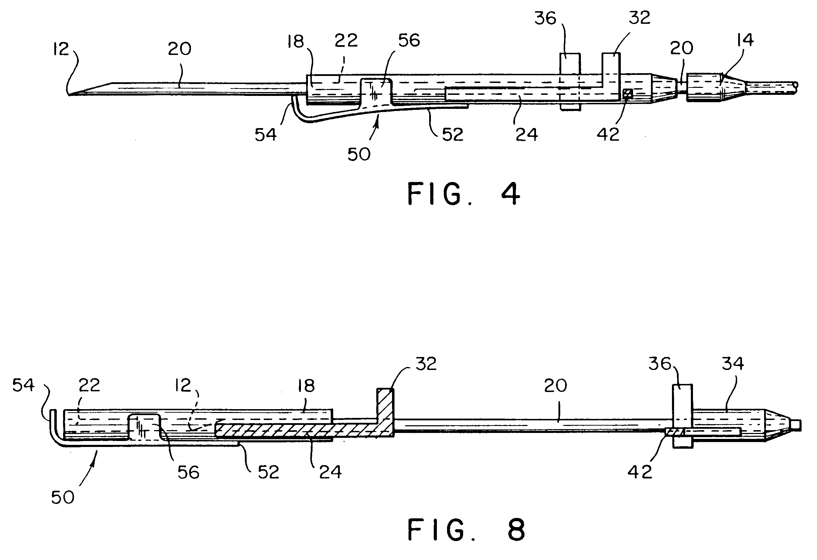

In the form of Figs. 1 and 2, and as seen in Fig. 4, the blocking plate 54 normally bears against needle 20 and thus is flexed against its initial curve as fabricated, as seen in Fig. 3, to maintain a spring force bias at all times toward shifting transversely of the tubular member 18.

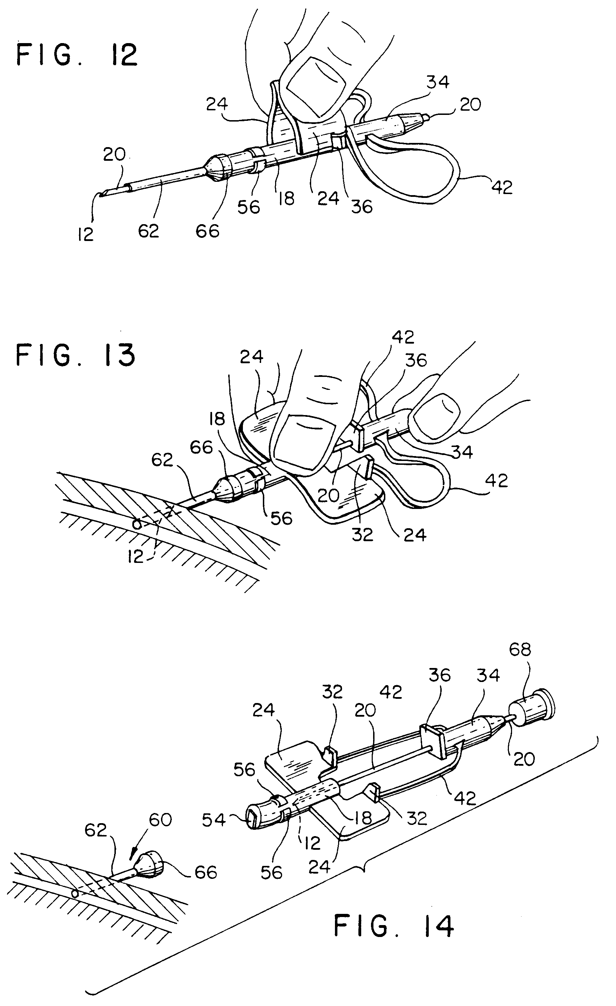

Assembled and ready for use, the needle 20 is initially slightly advanced by forward movement of base member 30, the needle sliding forwardly in guide member 10, the base member flange 36 moving into the recess 28 forwardly of the wing flanges 32. Thereupon, the wings 24 are flexed upwardly and gripped by opposing fingers to substantially a vertical position thereby to hold firmly the needle for insertion into tissue. While pertaining to a modified form of the invention, Figs. 12 and 13 illustrate the gripping action applicable to all forms of the invention which utilize wings 24 for needle insertion.

With the device so held, as in Fig. 12, the guide member, manipulating member, and needle move as a unit in inserting the needle into the skin. After needle insertion as desired, the wings are released to lie substantially flat as in Fig. 13 and be free of the interfering relationship between flanges 32 and with flange 36 of the manipulating member 30.

Upon completion of the intravenous or other technique, to withdraw the needle from the biological tissue, the wings 24 are held generally against the skin with the fingers of one hand while the opposite hand is used to grasp base member 34, bonded to needle 20, and pull it away from the skin puncture site in a proximal direction.

In so doing, the needle 20 slides rearwardly in passageway 22 , and in accordance with the invention, the interconnecting straps 42 are dimensioned to permit sufficient proximal movement of manipulating member 30 so as to withdraw the sharpened tip 12 of the needle fully into passageway 22 of the guide member 10, but not so far when fully extended as to permit the needle to be withdrawn from or even closely approach the proximate end of passageway 22 at recess 28, as clearly seen in Figs. 7 and 8.

Upon withdrawal of the needle tip into the guide member, in the form of the invention in Figs. 1-4, and Fig. 11, spring 50 is no longer restrained, and blocking plate 54 thereof snaps across the distal end of passageway 22 to positively prevent any hazardous egress of the tip whatsoever, as seen in Fig. 8. As is evident, the configuration of plate 54 will control whether the entire passageway is blocked, or merely sufficient to prevent access to the contaminated needle tip. In like manner, the axial location of the spring guard 50 on tubular portion 18 will control just how close the plate 54 will be to the distal end of the passageway. In any event, under any circumstance, the contaminated needle tip 12 is fully shielded and the tissue-puncturing distal length of needle 20 is enclosed within the guide member 18.

It will be recalled that the guide member 10 and indeed the entire operative assembly is injection molded in one preferred embodiment from suitable polymeric material, as nylon, polyvinyl chloride, polypropylene or the like.

Depending on the characteristics of the plastic material as well as dimensional parameters of the wall of tubular portion 18 and the strength of guard spring 50, withdrawal of the needle 20 well into the passageway and thereby removal of the radial support for the tube provided by the needle may permit transverse flexure of the tubular portion 18 to secure further the needle point from unwanted access or egress.

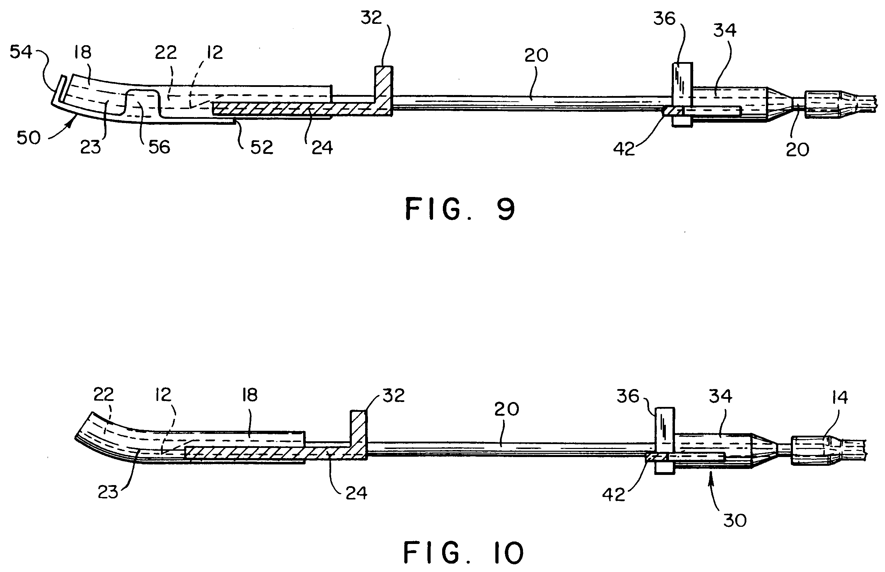

This aspect of the invention is seen in Fig. 9 which corresponds to Fig. 8 with the needle withdrawn, but wherein the combination of spring strength and flexure characteristics of the plastic tube cause the same to bend laterally or transversely, whereby potential egress of the needle tip from the passageway is blocked not only by the plate 54 but also primarily by the curve in tubular portion 18. In this regard it is further noted that the conventional bevel of the needlepoint at 12 is oriented as shown in Fig. 9 on assembly with respect to the spring that any advancing movement of the needle in the bore passageway 22 would urge the point to bite into the bore sidewall 23 and be stopped and held thereby. In this form, the blocking plate 54 is optional and may be eliminated, keeping the spring otherwise to apply transverse pressure to the tube end.

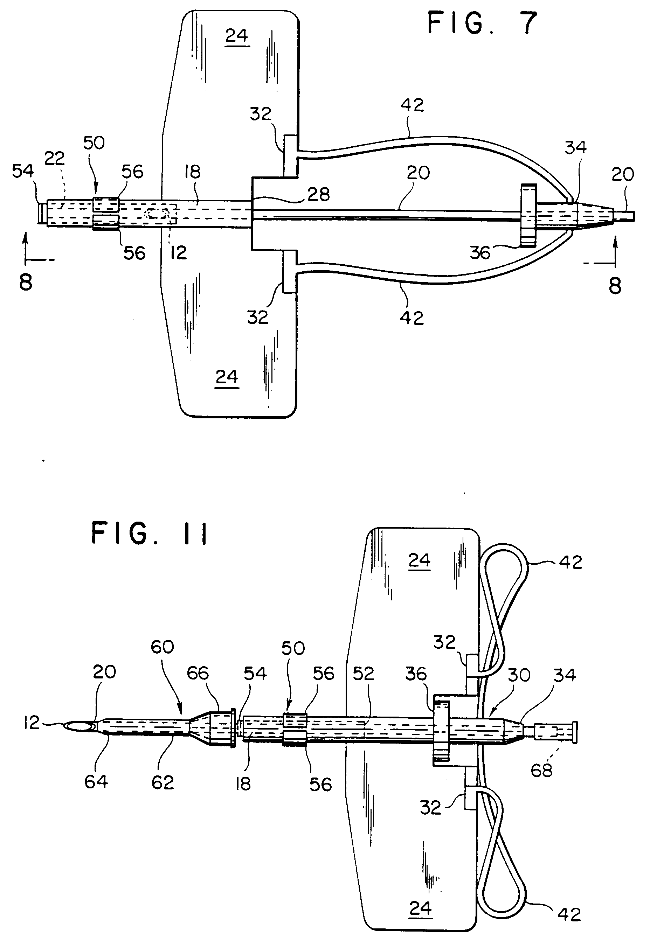

A further modification of this concept, which however is outside the scope of the claimed invention, is shown in Fig. 10 wherein the distal end of tubular portion 18 is initially formed from a plastic with shape memory characteristics and having an unstressed curvature as shown. Thus, the spring-like forces associated with the plastic component causing it to resume its original curved shape, which it had prior to assembly with needle cannula 20, result in the deflection of the unsupported distal end of tubular portion 18 following needle withdrawal therethrough whereby the needle tip 12 cannot emerge once withdrawn but bites into the curved tube wall 23 of passageway 22 to be held thereby if needle advance in the distal direction starts to occur.

Accordingly, in any form of the invention, the needle distal end section and especially the contaminated point are fully received within passageway 22 of guide member 10 and cannot be exposed therefrom whether distally or proximally, thereby obviating health hazards therefrom.

Figure 11 illustrates a further form of the invention in which the cooperating guide member, manipulating member, interconnecting, and interengaging elements are as in Fig. 1-4, including the spring-urged guard 50, but wherein the same is particularly adapted for introduction of a catheter into a vein or the like. To this end, an over-the-needle intravenous catheter 60 is carried by the needle 20, which punctures the skin, the distal end of the soft catheter 60 placed into a blood vessel, and the entire emplacement assembly withdrawn leaving the catheter in place. A major portion 62 of the catheter fits snugly as a sleeve on the needle 20, terminating short of the beveled needle tip 12. The sleeve portion is preferably tapered at its distal end at 64 for ease of insertion. The proximal end of the catheter is a hub 66 enlarged to facilitate use as by connection to feeding or intravenous devices, and may be rather flexible or somewhat rigid.

In use, the assembly is operated as described in connection with Figs. 1-4, and upon withdrawal of the needle, the distal end of the catheter remains implanted in the tissue, while the needle is safely captured as aforesaid. In like manner, the capture modes of Figs. 9 and 10 may be employed as well as that of Fig. 1.

In connection therewith, in order to determine proper needle location with respect to a blood vessel, the proximal end 14 of needle 20 instead of being connected to medical tubing is provided with a transparent or translucent vented chamber 68, whereby arrival of blood therein can be readily observed, after which the assembly is removed as described.

If desired, the blocking plate 54 of the spring 50 may be repositioned, and employed to bear against the hub 66 of the catheter instead of against the needle cannula proper, thereby retaining the catheter in association with the assembly. When the needle is withdrawn, radial support for the spring-biased plate 54 against hub 66 is removed, and the needle, guide member and manipulating member assembly can be taken away, the catheter 60 thereby becoming free to remain in the body.

In Figs. 12-14, the invention in the form illustrated in Fig. 11 for intravenous catheter placement is shown in perspective view. These figures further illustrate the preferred method of using the device for needle insertion, catheter placement, and safe withdrawal and disposal. Note that catheter 62 fits snugly on needle cannula 20 and is easily removed from same after the desired position in the blood vessel is obtained.

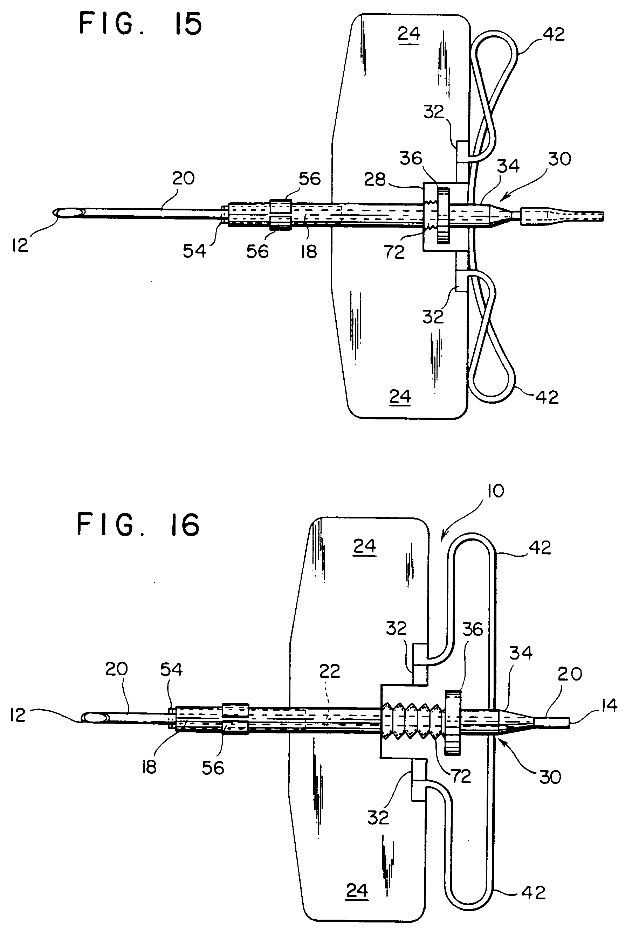

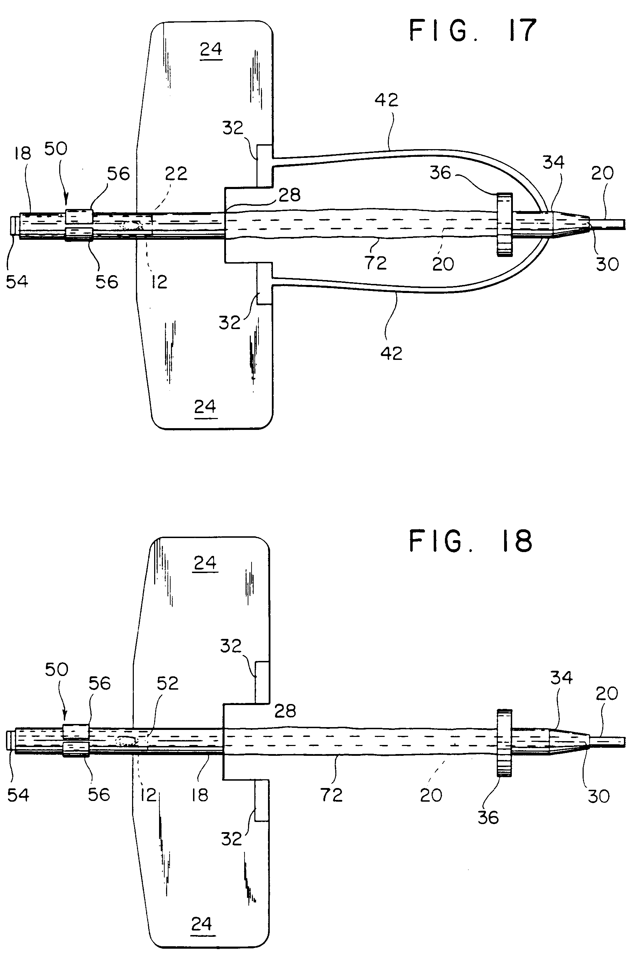

Yet another modification of the invention appears in Figs. 15-17 wherein a bellows-like expandable plastic sheath 72 is associated with the assembly of Figs. 1-4 or Fig. 11. As shown, sheath 72 is disposed in recess 28, connecting the proximal end of wings 24 with the flange 36 of the manipulating member 30, being affixed to both. Accordingly, as the needle 20 is withdrawn progressively as seen in Figs. 16 and 17, the sheath elongates and shields the incrementally exposed portion of needle 20, thereby providing further protection to healthcare workers against any hazardous contamination as tissue debris on middle or rearward reaches of needle 20 that may possibly have occurred.

A further variant appears in Fig. 18 wherein the expansible sheath is employed as the interconnecting means in lieu of the straps 40, which are eliminated. In this form of the invention, untoward proximal removal of the needle is prevented by the full extension of the sheath 72, which is dimensioned to be less than that required to permit the needle point to emerge from the proximal end of guide member 10 and puncture the sheath.

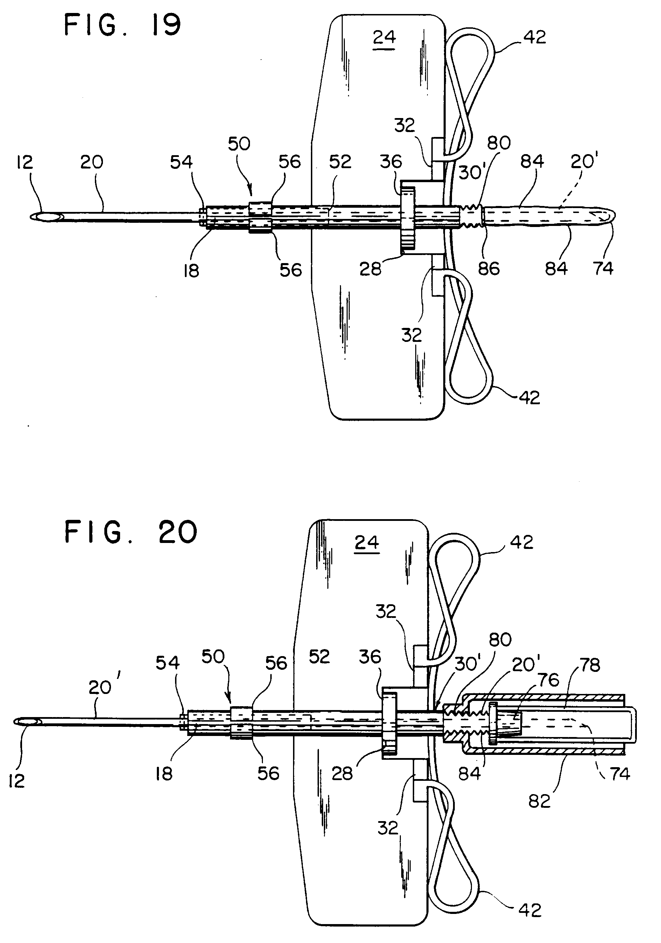

Figs. 19 and 20 illustrate a modification of the invention adapting the same for use with standard blood collection tubes. Thus the modified needle 20' therewith is a double-ended needle including proximal needle point 74 of usual nature for puncturing the rubber stopper 76 of standard blood collection tubes 78.

The modified base member 30' is provided with a proximal threaded extension 80 to which a conventional tube holder 82 is firmly threaded, thereby connecting the tube holder 82 fixedly with needle 20' bonded to manipulating member 30'. The tube holder also serves to shield medical personnel from the needle point 74 as well as aid in manipulation of the assembly in inserting the distal end 12 of the needle 20' into the flesh.

Additionally, a conventional flexible sheath of rubber or the like at 84 to facilitate taking sequential blood samples, encloses the proximal end of needle 20' and the point thereof and is secured to the threaded member 80 at 86.

Accordingly, upon insertion of a collection tube 78 into tube holder 82, the point 74 will puncture the rubber cap 76, sliding the sheath 84 in a distal direction as seen in Fig. 20, all as in current blood collection practices. As before, upon removal from tissue, the needle distal end 12 is captured and shielded in passageway 22.

A further modification of this invention is similar to that illustrated in Fig. 19 except that the proximal needle assembly is adapted to fit with the standard hypodermic syringe instead of the blood collection tube holder. In this case, as shown in Fig. 21, the manipulating member 30'' is designed to affix to a syringe. The proximal end 14' of needle 20'' is disposed within manipulating member 30''. The distal end 91 of syringe barrel 90 fits tightly with manipulating member 30'' as shown so that the interior chamber 92 of syringe barrel 90 is in fluid communication with the interior passageway of needle cannula 20''.

In the modifications shown in Figs. 19 and 21, needle insertion into tissue may be accomplished either while gripping wings 24 as described hereinabove or while holding the blood collection tube holder 82 or syringe barrel 90, respectively. When needle insertion is carried out while gripping the tube holder 82 or syringe barrel 90, the need for flanges 32 and 36 is obviated. The overall shape of the guide means and straps 42 may be modified as well to further provide design efficiency and to simplify manufacture.

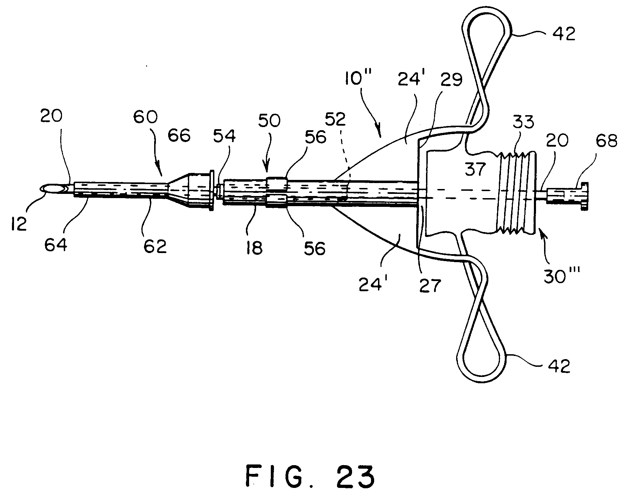

Figure 23 illustrates another preferred embodiment of the invention in which modified manipulating member 30''' provides a textured surface 33 and has a somewhat hourglass shape to facilitate finger gripping. Mechanical interaction between guide member 24' and manipulating member 30''', which latter is as before bonded to needle cannula 20, is confined to a separable abutment zone 27 between the members and by the interconnecting straps 42.

In this embodiment, member 30''' is gripped for insertion of needle tip 12 into biological tissue, during which insertion the distal end 37 of member 30''' abuts directly the proximal end 29 of guide member 10'' so that the two members move together during insertion. During needle withdrawal, leaving catheter 60 in desired position within the blood vessel, member 10'' is held in position at the skin puncture site while member 30''' is pulled away therefrom to the limit permitted by straps 42, thus retracting needle tip 12 into tubular segment 18 of member 10'', and the needle guard or shield means as at 54 (or the other means disclosed) operates as heretofore. In this form of the invention it will be seen that wings 24' are of reduced size and are not flexed in the manner previously shown, not having the interengagable abutments thereon.

It follows that it is a feature of the invention that, during needle insertion, the device could be used with either the separable non-interlocking interaction of the guide member and manipulating member as shown in Fig. 23, or the interengagable form as illustratively shown in Fig. 12.

In the basic form of the invention shown in Figs. 1-4, needle 20 is shown as received within a substantially cylindrical tubular member 18, and wherein further the spring guard 50 circularly embraces member 18 by clip legs 56.

Both the needle-receiving member as at 18 and the spring guard as at 50 may partake of other forms within the scope of the invention while functioning in the same position and reliable manner to effect fully safe operation and inaccessibility of the used needle point.

Thus, as seen in Figs. 3A and 24-27, a different embodiment of safety guard spring 145 is provided. Figure 3A illustrates in a perspective view the details of the improved spring 145. Spring 145 extends from its proximal end 148 along main length 151 to distal end 147. Along length 151 are disposed arms 146, which are designed to attach spring 145 to front section 141 of needle assembly 140. In one embodiment spring 145 is stainless steel and is cut and bent into the desired shape. Arms 146 are disposed somewhat perpendicularly to the main length 151, and each arm contains right angle cuts at edges 152 and 153. Further provided is a bend defining a line 154 between edges 153 and 155, and the bend is made such that a bent portion 156 of arm 146 is defined. The bottom edges 159 of portions 156 are disposed inwardly in the space between arms 146. Bent portion 156 remains integral to spring 145 through the continuum provided through bend 54.

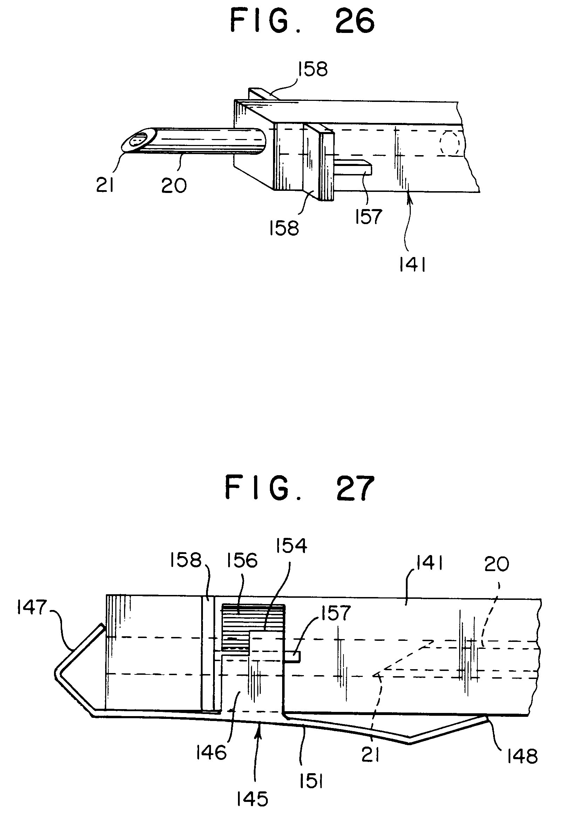

To provide the desired assembly of spring 145 to the front end section 141 may be provided with either protrusions or indentations, with which the spring arms 146 can be engaged irreversibly. In Figure 26 a perspective view of the distal end of extended front section 41 is shown with protrusions 157 and 158 extending outwardly from each side of tubular section 141, which in this form of the invention is of generally rectangular cross-sectional configuration.

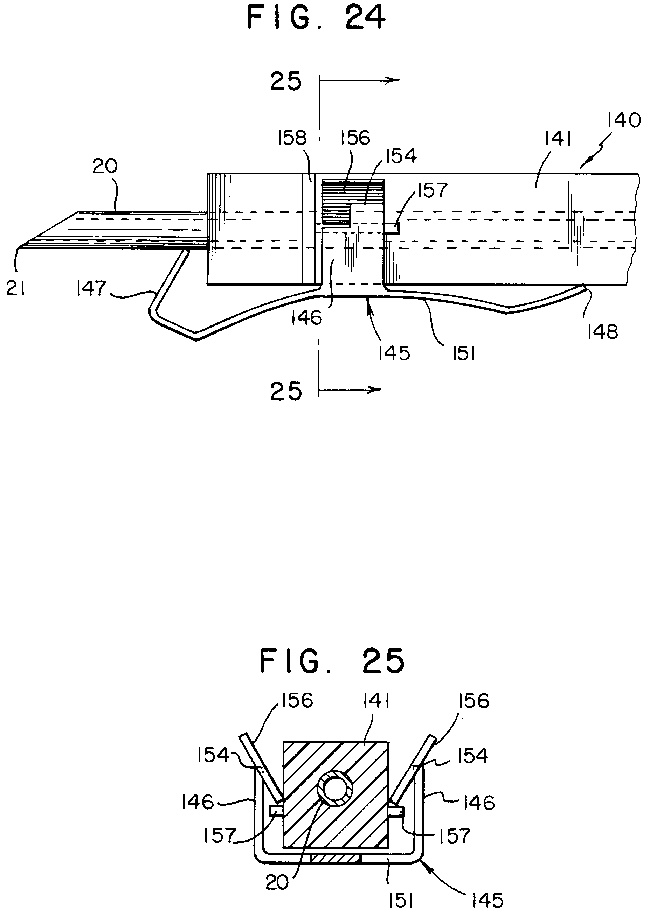

Figures 24 and 25 depict side and end views of the improved spring 145 and its assembly with front section 141. To assemble spring 145 with front section 141 in which needle 20 is disposed, spring 145 is simply pressed onto the bottom of section 141 until edges 159 of bent segments 156 engage or snap over protrusions 157. Because spring 145 is of a flexible metal, its segments 156 can be designed to dig into and thereby to affix securely to the opposite sides of the extended tubular section 141 and protrusions 157 as desired. This engagement of protrusions 157 by wing segments 156 is further illustrated in the cross sectional view shown in Figure 25. The force tending to cause spring arm segments 156 to dig downwardly into protrusions 157 is provided by the flexed spring 145 along its length 151 with forces in the opposing direction being exerted against needle 20 by spring distal end 147 and against extended front section 141 by proximal spring end 148. The spring force bias thus holds spring 145 securely onto front section 141 of needle assembly 140.

The vertical protrusions 158 further serve to prevent any distally directed movement of spring 45 because the proximal surfaces of protrusion 158 abut the distal edges of arms 146 (including bent segments 156). This feature serves therefore the important function of blocking the re-emergence of the contaminated needle point 21 after it is withdrawn in accordance with this invention as seen in Fig. 27.

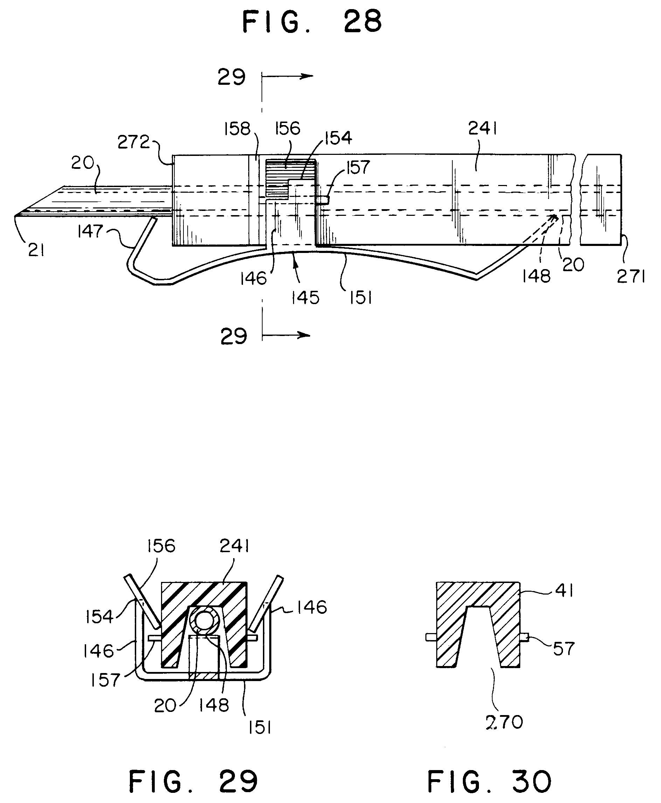

Figures 28-30 illustrate yet a further embodiment of needle assembly whose front body section 241 is provided with a groove 70 along its length from its proximal end 271 to its distal end 272. This groove provides a space into which needle 20 can be suitably disposed and held therein by the aforesaid spring 145. In this embodiment of the needle assembly, spring 145 can be affixed to the front section 241 so as to secure needle 20 in the groove. Thus spring 145 holds needle 20 in place by spring bias forces exerted against the needle at spring distal end 147 and spring proximal end 148 and exerted in the opposite direction through spring arms 146, which grip front section 241 by the engagement of bent arm segments 156 with protrusions 157 as described hereinabove. The cross sectional view of this embodiment of the needle assembly with needle 20 in grove 70 and with spring 145 in place is shown in Figure 29.

Groove 70 is an alternative to the tubular openings of front section 41 (Figs. 1-4) or 141 (Figs. 24-27) as described. The combination of groove 70 and the spring design of Fig. 3a affords certain economies of manufacture by simplifying the method of assembling needle 20 and thereby making the manufacturing process readily adaptable to existing, well known medical device assembly techniques. In addition, the combination of the groove 70 and spring 145 in no way diminishes either method of use of the safety needle assembly of this invention nor the protection it affords in shielding irreversibly the contaminated needle. In this latter regard, as the contaminated needle is withdrawn, the distal segment 147 of the spring snaps closed to cover the distal end of front section 241 and distal groove opening of groove 270, and comes to rest against the bottom surface of front section 241. The opposing forces in one direction exerted at distal end 147 and proximal end 148 and in the opposite direction through the engagement of spring arms 146 with protrusion 157 ensure safe securement of the needle.

Further the lengths of restraints 42 (Fig. 1) are selected so as to prevent the contaminated needle point 21 from being withdrawn in a proximal direction beyond some desired location along the extended length 51 of spring 45.

To manufacture the embodiment of the safety needle assembly of Figs. 28-30 with the benefits provided by the combination of the groove 70 of front section 241 and the improved spring 145, a series of steps may be followed in routine production. Thus, in one preferred form, the front section 241, the strap-like restraints 42 and base section 30 (Fig. 1) are formed in a mold of flexible material such as polyvinyl chloride. This component can be fed into a needle bonding within the base member opening, which extends from the distal end to the proximal end of base member 30. Section 241 can be subsequently moved into a position so that the distal segment of needle 20 is disposed into and lies within groove 70. Thereafter spring 145 is pressed over the engrooved needle and onto the protrusions 157 of section 241. These assembly steps provide a straight-forward manufacturing process with the advantage that needle 20 need not be "threaded" or worked through the tubular opening of Figs. 1-4, for example. The elimination of the need to thread the needle through the tubular opening of one embodiment of front section 241 has distinct economic advantages in certain needle assemblies of the present invention.

## Claims

1. A safety needle assembly which ensures single use and positively precludes re-use or hazardous exposure of contaminated needles, comprising
a needle (20) having a proximal end (14) and a distal portion terminating in a pointed end (12),
a body (18; 141; 241) for receiving and guiding the needle for sliding movement therein, and said body having a front surface having a passageway (22; 70) through which the needle distal portion is initially extended in a first direction for use outwardly of said front surface and retracted rearwardly after use in a second direction through said passageway into said body with said needle pointed end (12) no longer extending through said body front surface, and
a movable safety device (50, 145) cooperatively associated with the needle (20) and the body (18; 141; 241), characterised in that the device (50, 145) is operable immediately and positively to preclude any hazardous re-emergence of the contaminated needle distal portion through the front surface of the body upon retraction of the pointed end (12) of the needle (20) in the second direction through the passageway (22; 70) to a position no longer extending through the body front surface, thereby to retain the pointed end (12) within the body and with no portion thereof extending or projecting outwardly from said body through said front surface,
the safety device (50, 145) further including:
(1) a blocking flange (54, 147) disposed exteriorly of the body (18; 141; 241) adjacent the front surface of the body, the blocking flange being imperforate throughout its length, and
(2) a spring portion (50, 145) for bodily moving the blocking flange (54, 147) in a needle-blocking direction toward and across the front surface of the body into adjacent overlying relation to the passageway (20; 70) on the body front surface upon retraction of the needle (20) into the body, wherein in any position of the blocking flange (54, 147) along its length after moving in a needle-blocking direction across the longitudinal axis of the needle, the imperforate structure of the flange (54, 147) bars any needle movement past the flange,
said spring portion (50, 145) being disposed on one side of the body front surface, and the flange (54, 147) having no portion thereof extending beyond the front surface of the body on the other side thereof after moving to the needle blocking position, thereby to preclude any possible movement of the flange (54, 147) to unblock the passage against the bias of the spring,
whereby a used and contaminated needle (20) cannot have its pointed end (12) or adjacent distal portion projected outwardly from the body and thereby re-exposed for hazard, re-use or injury after its initial retraction into the body.

2. The safety needle assembly of claim 1 wherein the flange (54, 147) is disposed in spring-urged lateral engagement with the needle distal portion when the needle (20) is in its initial outwardly extended position relative to the body front surface.

3. The safety needle assembly of claim 2 further including interfering surfaces between the spring (50, 145) and the body (18; 141; 241) for limiting bodily movement of the imperforate flange (54, 147) by the spring in the needle blocking direction, thereby to maintain the flange (54, 147) in overlying blocking relation to the passageway (22; 70) when the needle (20) is retracted.

4. The safety needle assembly of claim 2 wherein the spring (50, 145) is disposed entirely exteriorly of the body (18; 141; 241), to facilitate assembly and inspection thereof and to ensure proper operation.

5. The safety needle assembly of claim 4 wherein the spring (50, 145) is a leaf spring secured to said body (18; 141; 241) and the blocking flange (54, 147) is a short, substantially planar end portion of the leaf spring.

6. The safety needle assembly of claim 4 wherein the blocking flange (54, 147) merges into a leaf spring portion which is disposed on one side of the body (18; 141; 241), with the leaf spring portion being disposed in spaced stressed relationship to the body when the blocking flange (54, 147) is in lateral engagement with the needle (20) when the latter is in its initial outwardly extending position.

7. The safety needle assembly of claim 4 wherein the spring includes gripping flanges (56; 146) engaged with the body and securely mounting the spring thereon.

8. The safety needle assembly of claim 1 further including a needle manipulating element (30) disposed rearwardly of the body (18; 141; 241) and secured to the needle (20), whereby manual movement of the manipulating element relative to the body (18; 141; 241) moves the needle (20) with respect to the body, and
structure (40) limiting separating movement of the body and the manipulating device to preclude withdrawal of the needle (20) from the body, the said structure permitting sufficient separating movement as to retract the distal end (12) of the needle (20) into the body.

9. The safety needle assembly of claim 8 wherein the structure (40) limiting movement is a flexible tether (42) connected between the body (18; 141; 241) and the needle manipulating element (30).

10. The safety needle assembly of claim 8 further including a flexible and extensible sleeve (72, Fig. 16) enclosing the needle between the body (18; 141; 241) and the needle manipulating element to preclude possible contamination from an exposed needle portion when the needle manipulating element is rearwardly separated from the body.

11. A method of immediately and positively precluding needlestick injury from a contaminated needle by ensuring single use thereof in an assembly having an elongated needle (20) with a pointed end (12), and a body (18; 141; 241) slidably receiving the needle and having a front surface through which the needle (20) extends for use and to or behind which it is retracted after use, characterised by the steps of:
providing a spring (50; 145) with a blocking flange (54; 147) which is imperforate over the entire length of the flange, and
affixing said spring (50; 145) to the body to dispose the flange (54; 147) adjacent the body front surface and spring-biassed against the needle (20) when extending from the body,
whereby upon retracting the needle (20) after use to bring its pointed end (12) flush with or behind the said front surface, the imperforate blocking flange (54; 147) is spring urged over the body front surface past the needle point to a needle blocking position, thereby to block any possibility of re-emergence of the needle (20) from the body (18; 141; 241);
wherein further the step of providing the spring (50; 145) and flange (54; 147) include providing the latter with a configuration such that no portion of the flange (54;147) extends beyond the body front surface, other than its connection to the spring (50; 145), after the flange has moved to its needle-blocking position, thereby to preclude any possible movement of the flange (54; 147) against the bias of the spring in a direction unblocking the needle (20),
whereby a used needle (20) cannot have its pointed end (12) or adjacent distal portion re-exposed for hazard and injury after initial retraction thereof into said body.

## Patentansprüche

1. Eine Sicherheitsspritzeneinrichtung, die den einmaligen Gebrauch gewährleistet und zwangsläufig die Wiederverwendung oder das gefährliche Freilegen der kontaminierten Nadeln ausschließt, weist auf:
eine Nadel (20) mit einem proximalen Ende (14) und einem distalen Abschnitt, der in einem spitzen Ende (12) endet,
einen Körper (18; 141; 241) zur Aufnahme und Führung der Nadel in einer gleitenden Bewegung darin und der Körper weist eine vordere Fläche mit einem Durchgang (22; 70) auf, durch den sich der distale Bereich der Nadel für den Gebrauch in eine erste Richtung aus der vorderen Fläche heraus erstreckt und nach dem Gebrauch in eine zweite Richtung durch den Durchgang in den Körper zurückgezogen ist, wobei sich das spitze Ende (12) der Nadel nicht mehr durch die vordere Fläche des Körpers erstreckt, und
eine bewegliche Sicherheitseinrichtung (50, 145), die mit der Nadel (20) und dem Körper (18; 141; 241) zusammenwirkt, dadurch gekennzeichnet, daß die Einrichtung (50, 145) sofort und zwangsläufig so betrieben wird, daß jedes gefährliche Wiederfreilegen des kontaminierten Abschnitts der Nadel durch die vordere Fläche des Körpers nach dem Zurückziehen des spitzen Endes (12) der Nadel (20) in die zweite Richtung durch den Durchgang (22; 70) in eine sich nicht mehr durch die vordere Fläche des Körpers erstreckende Position ausgeschlossen ist, wobei das spitze Ende (12) innerhalb des Körpers zurückgehalten wird und sich kein Abschnitt derselben aus dem Körper durch die vordere Fläche heraus erstreckt oder zeigt,
wobei die Sicherheitseinrichtung (50, 145) ferner umfaßt:
(1) einen blockierenden Ansatz (54, 147), der außerhalb des Körpers (18; 141; 241) in der Nähe der vorderen Fläche des Körpers angeordnet ist, wobei der blockierende Ansatz entlang seiner Länge unperforiert ist, und
(2) einen Federabschnitt (50, 145) zum Verschieben des blockierenden Ansatzes (54, 147) in eine Nadel-Blockierrichtung zu und über die vordere Fläche des Körpers in eine den Durchgang (20; 70) an der vorderen Fläche des Körpers in der Nähe abdeckende Position nach dem Zurückziehen der Nadel (20) in den Körper, worin in jeder Position des blockierenden Ansatzes (54, 147) entlang seiner Länge nach dem Verschieben in die Nadel-Blockierrichtung entlang der Längsachse der Nadel die unperforierte Struktur des Ansatzes (54, 147) jede Bewegung der Nadel hinter den Ansatz blockiert,
wobei der Federabschnitt (50, 145) auf der einen Seite der vorderen Fläche des Körpers angeordnet ist und der Ansatz (54, 147) keinen Abschnitt aufweist, der sich über die vordere Fläche des Körpers zu dessen andere Seite nach dem Verschieben in die Nadel-Blockierposition hinaus erstreckt, damit jede mögliche Bewegung des Ansatzes (54, 147) ausgeschlossen ist, um den Durchgang entgegen der Federvorspannung freizumachen,
wodurch das spitze Ende (12) oder der benachbarte distale Abschnitt einer benutzten und kontaminierten Nadel (20) sich nicht aus dem Körper heraus erstrecken kann und nach ihrem erstmaligen Zurückziehen in den Körper dadurch nicht wieder für ein Risiko, eine Wiederverwendung oder eine Verletzung freigelegt werden kann.

2. Die Sicherheitsnadeleinrichtung nach Anspruch 1, in der der Ansatz (54, 147) mit dem distalen Abschnitt der Nadel in einem federnd vorgespannten lateralen Eingriff angeordnet ist, wenn sich die Nadel in ihrer nach draußen erstreckenden Anfangsposition bezüglich der vorderen Fläche des Körpers befindet.

3. Sicherheitsnadeleinrichtung nach Anspruch 2, weiterhin umfassend Interferenzflächen zwischen der Feder (50, 145) und dem Körper (18; 141; 241) zur Begrenzung des Verschiebens des unperforierten Ansatzes (54, 147) durch die Feder in die Nadel-Blockierrichtung, wodurch der Ansatz (54, 147) in einer abdeckenden Blockierstellung zu dem Durchlaß (22; 70) gehalten wird, wenn die Nadel (20) zurückgezogen wird.

4. Die Sicherheitsnadeleinrichtung nach Anspruch 2, bei der die Feder (50, 145) völlig außerhalb des Körpers (18; 141; 241) angeordnet ist, um den Zusammenbau und die Inspektion derselben zu erleichtern und einen sicheren Betrieb zu gewährleisten.

5. Die Sicherheitsnadeleinrichtung nach Anspruch 4, bei der die Feder (50, 145) eine an dem Körper (18; 141; 241) gesicherte Blattfeder ist und der blockierende Ansatz (54, 147) ein kurzer, im wesentlichen ebener Endabschnitt der Blattfeder ist.

6. Die Sicherheitsnadeleinrichtung nach Anspruch 4, bei der der blockierende Ansatz (54, 147) in einen Blattfederabschnitt übergeht, der auf der einen Seite des Körpers (18; 141; 241) angeordnet ist, wobei der Blattfederabschnitt zu dem Körper mit Zwischenraum in einer gespannten Beziehung zu dem Körper angeordnet ist, wenn der Blockieransatz (54, 147) mit der Nadel (20) in lateralem Eingriff ist, wenn die letztere sich in ihrer nach draußen erstreckenden Anfangsposition befindet.

7. Die Sicherheitsnadeleinrichtung nach Anspruch 4, bei der die Feder Spannansätze (56; 146) umfaßt, die mit dem Körper in Eingriff sind und die Feder daran sicher befestigen.

8. Die Sicherheitsnadeleinrichtung nach Anspruch 1 ferner umfassend ein Nadelmanipulationselement (30), das an dem Körper (18; 141; 241) rückwärtig angeordnet ist und an der Nadel (20) befestigt ist, wodurch eine manuelle Bewegung des Manipulationselementes relativ zu dem Körper (18; 141; 241) die Nadel (20) bezüglich des Körpers verschiebt, und
eine Struktur (40), die das Auseinanderbewegen des Körpers und der Manipulationseinrichtung begrenzt, um das Zurückziehen der Nadel (20) aus dem Körper auszuschließen, wobei die Struktur ein ausreichendes Auseinanderbewegen erlaubt, so daß das distale Ende (12) der Nadel (20) in den Körper zurückgezogen werden kann.

9. Die Sicherheitsnadeleinrichtung nach Anspruch 8, bei der die die Bewegung begrenzende Struktur (40) ein flexibles Seil (42) ist, das den Körper (18; 141; 241) und das Nadelmanipulationselement (30) miteinander verbindet.

10. Die Sicherheitsnadeleinrichtung nach Anspruch 8, ferner umfassend eine flexible und ausdehnbare Hülse (72, Figur 16), die die Nadel zwischen dem Körper (18; 141; 241) und dem Nadelmanipulationselement einschließt, um eine mögliche Kontamination von einem freiliegenden Nadelabschnitt auszuschließen, wenn das Nadelmanipulationselement rückwärtig von dem Körper getrennt ist.

11. Ein Verfahren zum sofortigen und zwangsläufigen Ausschließen einer Nadelstichverletzung mit einer kontaminierten Nadel durch das Sicherstellen des einmaligen Gebrauchs der Nadel in einer Einrichtung, die eine langgestreckte Nadel (20) mit einem spitzen Ende (12) und einen Körper (18; 141; 241) umfaßt, der die Nadel verschiebbar aufnimmt und eine vordere Fläche aufweist, durch die sich die Nadel (20) beim Gebrauch erstreckt und zu der oder hinter die die Nadel nach dem Gebrauch zurückgezogen wird, gekennzeichnet durch die folgenden Schritte:
Vorsehen einer Feder (50; 145) mit einem blockierenden Ansatz (54; 147), der über die gesamte Länge des Ansatzes unperforiert ist, und
Anbringen der Feder (50; 145) an dem Körper, um den Ansatz (54; 147) in der Nähe der vorderen Fläche des Körpers und federnd vorgespannt gegen die Nadel (20) anzuordnen, wenn sich diese aus dem Körper heraus erstreckt,
wodurch nach dem Zurückziehen der Nadel (20) nach dem Gebrauch, um deren spitzes Ende (12) mit der vorderen Fläche zur Deckung zu bringen oder hinter die vordere Fläche zu bringen, der unperforierte blockierende Ansatz (54; 147) federnd über die vordere Fläche des Körpers hinter die Nadelspitze in eine Nadel-Blockierposition gedruckt wird, wodurch das Wiederfreilegen der Nadel (20) aus dem Körper (18; 141; 241) auf jeden Fall verhindert wird;
wobei der Schritt des Vorsehens der Feder (50; 145) und des Ansatzes (54; 147) ferner das Vorsehen des letzteren mit einer Konfiguration derart umfaßt, daß sich kein Abschnitt des Ansatzes (54; 147) über die vordere Fläche des Körpers hinaus erstreckt, anders als dessen Verbindung mit der Feder (50; 145), nachdem sich der Ansatz in seine Nadel-Blockierposition bewegt hat, wodurch jede mögliche Bewegung des Ansatzes (54; 147) entgegen der Federspannung in eine Richtung verhindert wird, in der die Nadel (20) freigegeben wird,
wodurch das spitze Ende (12) oder der benachbarte distale Abschnitt einer benutzten Nadel (20) nach dem anfänglichen Zurückziehen in den Körper nicht wieder für ein Risiko und eine Verletzung freigelegt werden kann.

## Revendications

1. Ensemble à aiguille de sécurité, assurant une seule utilisation et empêchant positivement une nouvelle utilisation ou une exposition dangereuse d'aiguilles contaminées, comprenant :
une aiguille (20) possédant une extrémité proximale (14) et une partie distale se terminant par une extrémité pointue (12),
un corps (18 ; 141 ; 241) destiné à loger et guider l'aiguille afin qu'elle puisse coulisser dans le corps, celui-ci ayant une surface avant qui possède un passage (22 ; 70) dans lequel la partie distale de l'aiguille avance initialement dans un premier sens afin qu'elle soit utilisée à l'extérieur de la surface avant et qu'elle recule vers l'arrière, après utilisation dans un second sens, dans le passage et dans le corps, afin que l'extrémité pointue (12) de l'aiguille ne dépasse pas de la surface avant du corps, et
un dispositif mobile de sécurité (50, 145) associé à l'aiguille (20) et au corps (18 ; 141 ; 241) avec lequel il coopère, caractérisé en ce que le dispositif (50 ; 145) empêche immédiatement et positivement toute nouvelle sortie dangereuse de la partie distale de l'aiguille contaminée par la surface avant du corps après recul de l'extrémité pointue (12) de l'aiguille (20) dans le second sens dans le passage (22 ; 70) en position qui ne dépasse pas la surface avant du corps, afin que l'extrémité pointue (12) soit retenue dans le corps, sans partie s'étendant à l'extérieur du corps ou dépassant de celui-ci par la surface avant,
le dispositif de sécurité (50 ; 145) comprenant en outre :
(1) un flasque de blocage (54 ; 147) disposé à l'extérieur du corps (18 ; 141 ; 241), adjacent à la surface avant du corps, le flasque de blocage n'étant pas perforé sur toute sa longueur, et
(2) une partie à ressort (50 ; 145) destinée au déplacement global du flasque de blocage (54, 147) dans le sens de blocage d'aiguille vers la surface avant du corps et transversalement à celle-ci en position de recouvrement sous forme adjacente du passage (20 ; 70) à la surface avant du corps après recul de l'aiguille (20) dans le corps, si bien que, dans toute position du flasque de blocage (54 ; 147) sur sa longueur après déplacement dans le sens de blocage d'aiguille transversalement à l'axe longitudinal de l'aiguille, la structure non perforée du flasque (54 ; 147) empêche tout déplacement de l'aiguille au-delà du flasque,
la partie de ressort (50 ; 145) étant disposée d'un premier côté de la surface avant du corps et le flasque (54 ; 147) n'ayant aucune partie qui dépasse au-delà de la surface avant du corps de l'autre côté de celui-ci après déplacement de la position de blocage d'aiguille, si bien que tout déplacement possible du flasque (54 ; 147) destiné à débloquer le passage malgré la force de rappel du ressort est empêché,
si bien qu'une aiguille utilisée et contaminée (20) ne peut pas avoir son extrémité pointue (12) ou sa partie distale adjacente qui dépasse à l'extérieur du corps et ainsi est exposée à nouveau en présentant un danger, en permettant une nouvelle utilisation ou en permettant une blessure après le recul initial dans le corps.

2. Ensemble à aiguille de sécurité selon la revendication 1, dans lequel le flasque (54 ; 147) est disposé afin qu'il coopère latéralement sous l'action d'un ressort avec la partie distale de l'aiguille lorsque l'aiguille (20) est dans sa position initiale dépassant vers l'extérieur par rapport à la surface avant du corps.

3. Ensemble à aiguille de sécurité selon la revendication 2, comprenant en outre des surfaces de contact placées entre le ressort (50 ; 145) et le corps (18 ; 141 ; 241) et destinées à limiter le déplacement global du flasque non perforé (54 ; 147) par le ressort dans le sens de blocage d'aiguille, si bien que le flasque (54 ; 147) est maintenu en position de blocage et de recouvrement du passage (22 ; 70) lorsque l'aiguille (20) a reculé.

4. Ensemble à aiguille de sécurité selon la revendication 2, dans lequel le ressort (50 ; 145) est placé entièrement à l'extérieur du corps (18 ; 141 ; 241) afin que le montage et l'inspection du corps soient facilités et que le fonctionnement normal soit assuré.

5. Ensemble à aiguille de sécurité selon la revendication 4, dans lequel le ressort (50 ; 145) est un ressort à lame fixé au corps (18 ; 141 ; 241) et le flasque de blocage (54 ; 147) est une courte partie d'extrémité pratiquement plane du ressort à lame.

6. Ensemble à aiguille de sécurité selon la revendication 4, dans lequel le flasque de blocage (54 ; 147) se raccorde à la partie de ressort à lame qui est placée d'un côté du corps (18 ; 141 ; 241), la partie de ressort à lame étant placée à distance du corps et sous une contrainte la rappelant vers le corps lorsque le flasque de blocage (54 ; 147) est en contact latéral avec l'aiguille (20) et celle-ci est dans sa position initiale dépassant vers l'extérieur.

7. Ensemble à aiguille de sécurité selon la revendication 4, dans lequel le ressort comporte des flasques de saisie (56 ; 146) coopérant avec le corps et assurant un montage fixe du ressort sur le corps.

8. Ensemble à aiguille de sécurité selon la revendication 1, comprenant en outre un élément (30) de manipulation d'aiguille placé en arrière du corps (18 ; 141 ; 241) et fixé à l'aiguille (20) afin qu'un déplacement manuel de l'élément de manipulation par rapport au corps (18 ; 141 ; 241) déplace l'aiguille (20) par rapport au corps, et
une structure (40) limitant le mouvement de séparation du corps et du dispositif de manipulation pour empêcher l'extraction de l'aiguille (20) du corps, la structure permettant un mouvement suffisant de séparation pour que l'extrémité distale (12) de l'aiguille (20) recule dans le corps.

9. Ensemble à aiguille de sécurité selon la revendication 8, dans lequel la structure (40) qui limite le déplacement est un lien flexible (42) raccordé entre le corps (18 ; 141 ; 241) et l'élément de manipulation d'aiguille (30).

10. Ensemble à aiguille de sécurité selon la revendication 8, comprenant en outre un manchon flexible et extensible (72, figure 16) qui entoure l'aiguille entre le corps (18 ; 141 ; 241) et l'élément de manipulation d'aiguille pour empêcher une contamination possible par une partie exposée d'aiguille lorsque l'élément de manipulation d'aiguille est séparé du corps vers l'arrière.

11. Procédé destiné à empêcher immédiatement et positivement une blessure par une pointe d'aiguille contaminée par détermination certaine d'une utilisation unique de l'aiguille dans un ensemble ayant une aiguille allongée (20) possédant une extrémité pointue (12), et un corps (18 ; 141 ; 241) logeant de manière coulissante l'aiguille et ayant une surface avant par laquelle l'aiguille (20) dépasse lorsqu'elle est utilisée et vers laquelle et derrière laquelle elle recule après utilisation, caractérisé par les étapes suivantes :
la disposition sur un ressort (50 ; 145) d'un flasque de blocage (54 ; 147) qui n'est pas perforé sur toute sa longueur, et
la fixation du ressort (50 ; 145) sur le corps afin que le flasque (54 ; 147) soit adjacent à la surface avant du corps et rappelé élastiquement contre l'aiguille (20) lorsqu'elle dépasse du corps,
si bien que, après recul de l'aiguille (20) lorsqu'elle a été utilisée, afin que son extrémité pointue (12) revienne au niveau de la surface avant ou derrière cette surface, le flasque non perforé de blocage (54 ; 147) est rappelé élastiquement sur la surface avant du corps au-delà de la pointe de l'aiguille vers une position de blocage d'aiguille de sorte que toute possibilité de nouvelle sortie de l'aiguille (20) du corps (18 ; 141 ; 241) est empêchée,
dans lequel l'étape de disposition du ressort (50 ; 145) et du flasque (54 : 147) comporte en outre la réalisation du flasque avec une configuration telle qu'aucune partie du flasque (54 ; 147) ne dépasse au-delà de la surface avant du corps, autrement qu'à sa connexion au ressort (50 ; 145), après que le flasque a été déplacé vers sa position de blocage d'aiguille, si bien que tout déplacement possible du flasque (54 ; 147) dans le sens antagoniste de la force de rappel du ressort, dans le sens du déblocage de l'aiguille (20), est empêché,
si bien qu'une aiguille utilisée (20) ne peut pas avoir son extrémité pointue (12) ou sa partie distale adjacente à nouveau exposée de manière dangereuse, pouvant provoquer une blessure, après son recul initial dans le corps.
